**Europäisches Patentamt**

**European Patent Office**

**Office européen des brevets**

(19)

(11) Veröffentlichungsnummer: **0 318 773 B1**

## EUROPÄISCHE PATENTSCHRIFT

(12)

(45) Veröffentlichungstag der Patentschrift:
16.10.91 Patentblatt 91/42

(21) Anmeldenummer: 88119200.9

(22) Anmeldetag: 18.11.88

(51) Int. Cl.⁵: **A61K 31/195**, A61K 33/04,
A61K 31/575, // (A61K31/575,
31:195), (A61K33/04, 31:575,
31:195)

(54) Pharmazeutische Zubereitung zur Cholelitholyse.

(30) Priorität: 20.11.87 DE 3739401

(43) Veröffentlichungstag der Anmeldung:
07.06.89 Patentblatt 89/23

(45) Bekanntmachung des Hinweises auf die
Patenterteilung:
16.10.91 Patentblatt 91/42

(84) Benannte Vertragsstaaten:
AT BE CH DE FR GB IT LI LU NL SE

(56) Entgegenhaltungen:
DE-A- 2 936 070
FR-A- 2 470
GB-A- 2 036 558
US-A- 1 714 430

(56) Entgegenhaltungen:
CHEMICAL ABSTRACTS, Band 103, Nr.21, 25.
November 1985, Seite 59, Nr. 171825x, Columbus, Ohio, US; B.F. SMITH et al.:
"Identification of gallbladder mucin-bilirubin
complex in human cholesterol gallstone matrix. Effects of reducing agents on in vitro
dissolution of matrix and intact gallstones", &
J. CLIN. INVEST. 1985, 76(2), 439-45

(73) Patentinhaber: Leuschner, Ulrich, Prof. Dr.
Klaus-Groth-Strasse 10
W-6000 Frankfurt/Main 1 (DE)

(72) Erfinder: Leuschner, Ulrich, Prof. Dr.
Klaus-Groth-Strasse 10
W-6000 Frankfurt/Main 1 (DE)

(74) Vertreter: Patentanwälte Deufel, Hertel,
Lewald
Isartorplatz 6 Postfach 26 02 47
W-8000 München 26 (DE)

Anmerkung: Innerhalb von neun Monaten nach der Bekanntmachung des Hinweises auf die Erteilung
des europäischen Patents kann jedermann beim Europäischen Patentamt gegen das erteilte europäische Patent Einspruch einlegen. Der Einspruch ist schriftlich einzureichen und zu begründen. Er gilt
erst als eingelegt, wenn die Einspruchsgebühr entrichtet worden ist (Art. 99(1) Europäisches Patentübereinkommen).

EP 0 318 773 B1

## Beschreibung

Die Erfindung betrifft neue pharmazeutische Zubereitung, sowie die Verwendung dieser Zubereitung zur Cholelitholyse (Auflösung von Gallensteinen).

Gallensteine treten beim Menschen im Bereich der Gallenblase und der Gallengänge als Cholesterinsteine oder als Pigmentsteine auf. Zu ihrer Entfernung wurden Methoden entwickelt, bei denen — abgesehen von der Steinentfernung mit operativen und endoskopischen Verfahren — mit chemischen und physikalischen Mitteln gearbeitet wurde.

So wurde z.B. das Verfahren der Steinzertrümmerung durch Anwendung physikalischer Stoßwellen, das ursprünglich für Nierensteine entwickelt und ausschließlich dafür eingesetzt wurde, in jüngster Zeit auch für Gallensteine eingesetzt.

Daneben wurde auch versucht, Gallensteine auf chemischem Wege aufzulösen. Dabei ist zwar die Auflösung von Cholesterinsteinen weitgehend gelungen, Schwierigkeiten bereitet jedoch das Auflösen von Pigmentsteinen. Hierzu sind Versuche bekannt, den Calciumanteil (Calciumbilirubinat, Calciumcarbonat) mit Komplexbildnern, insbesondere Chelatkomplexbildnern herauszulösen und so das Gefüge des Steins zu zerstören, wobei er in Gallengrieß übergeht und ausgeschieden werden kann. Geeignete Komplexbildner sind z.B. Ethylendiamintetraessigsäure (EDTA) oder ihr Dinatriumsalz (EDTA-Na$_2$).

Aus Chemical Abstracts, Band 103, Nr. 21, 25. November 1985, Seite 59, Nr. 171825x ist die beschleunigte Auflösung von Gallensteinen in wäßrigen Pufferlösungen, die das als Disulfidbrückenspalter wirkende N-Acetylcystein enthalten, bekannt. In der DE-A 2936070 wird eine Gallenwegspüllösung beschrieben, die in wäßriger Pufferlösung Natrium-Chenodesoxycholat als Emulgator und EDTA-tri-Carnosinsalz als Komplexbildner enthalten kann. Gemäß GB-A-2036558 wird zur Lyse von Cholesterin-Gallenblasensteinen eine pharmazeutische Zusammensetzung eingesetzt, die als Wirksubstanz eine als Emulgator wirkende Gallensäure enthält.

Die vorstehend erläuterten bekannten Verfahren zur Gallensteinentfernung sind aus vielerlei Gründen unbefriedigend. Die Anwendung des Stoßwellenverfahrens scheitert in vielen Fällen an den außerordentlich hohen Kosten, die bei der Anschaffung der dafür erforderlichen Geräte sowie für deren Wartung und Unterhalt anfallen.

Zur chemischen Auflösung wurde noch keine pharmazeutische Zubereitung gefunden, mit der insbesondere Pigmentsteine hinreichend schnell aufgelöst werden können. So hat es sich z.B. gezeigt, daß Patienten in schwierigen Fällen von Cholelithiasis — bei denen 100 Steine oder mehr in den Gallengängen und in der Gallenblase anzutreffen sind- unter Umständen mehr als 5 l EDTA-Na$_2$-Lösung über eine Sonde in die Gallenwege eingeführt werden müssen, wozu 50 Behandlungstage oder mehr erforderlich sein können. Obwohl Nebenwirkungen bei der vorstehend beschriebenen Applizierung von EDTA-Na$_2$-Lösung nur in Einzelfällen beobachtet wurden, erscheint es jedoch aus grundsätzlichen Überlegungen wünschenswert, insbesondere in Fällen von schwerer Cholelithiasis die Behandlungszeiten erheblich zu verkürzen.

Es ist deshalb eine Aufgabe der Erfindung, eine pharmazeutische Zubereitung anzugeben, mit der insbesondere Pigmentgallensteine schneller als bisher aufgelöst werden können. Eine weitere Aufgabe der Erfindung ist es, die Behandlungszeiten und die Behandlungskosten insbesondere auch in Fällen von schwerer Cholelithiasis zu verkürzen und die Belastung des Patienten durch die zugeführten Lösemittel und durch die eingeführte Sonde zu verringern. Daneben ist es auch eine Aufgabe der Erfindung, eine pharmazeutische Zubereitung zur Auflösung von Gallensteinen, insbesondere Pigmentgallensteinen anzugeben, die einfach und kostengünstig herzustellen ist.

Die Lösung dieser Aufgabe erfolgt erfindungsgemäß mit einer pharmazeutischen Zubereitung gemäß Anspruch 1 und der Verwendung gemäß Anspruch 7.

Die Erfindung geht davon aus, daß die Auflösung von Pigmentgallensteinen durch einen den Calciumanteil bindenden Komplexbildner vermutlich deshalb langsam voranschreitet, weil Pigmentsteine eine organische Matrix aufweisen, die die abzubauenden Calciumverbindungen umhüllen und so den Zutritt des Komplexbildners zu den Calciumverbindungen hemmen. Demnach müßte es beim Abbau von Gallensteinen, insbesondere von Pigmentgallensteinen darauf ankommen, auch die organische Matrix aufzulösen, die im wesentlichen aus Eiweißverbindungen, zum Teil aus zuckerhaltigen Eiweißverbindungen besteht, und die Auflösungsprodukte kolloidal in Lösung zu halten bzw. zu emulgieren.

Auf der Suche nach hierfür geeigneten Substanzen wurde überraschend gefunden, daß Pigmentsteine auch von Substanzen aufgelöst werden, die als Disulfidbrückenspalter (DSS) bekannt sind. Geeignete DSS sind z.B. Cystein, N-Acetylcystein (NAC), Cysteamin, D-Penecillamin (DPA), Mercaptopropionglycin, Dithiothreitol, Glutathion (GSH), Methionin und anorganische Sulfide und Hydrogensulfide wie Me$_2$S und MeHS, wobei Me die Bedeutung Na, K oder NH$_4$ haben kann, und Gemische davon.

Die DSS werden zusammen mit anderen Wirkstoffen in Form von pharmazeutischen Zubereitungen eingesetzt, die die DSS oder Gemische davon sowie die anderen Wirkstoffe in wäßriger Lösung gelöst enthalten.

2

Die pharmazeutische Zubereitung wird mit Hilfe einer Sonde oder eines Katheters direkt in die Gallenwege appliziert, so daß die Gallensteine davon umspült werden. Es erfolgt ein Abbau der Steine zu grießartigen Abbauprodukten (Gallengrieß), die dann über die Faeces ausgeschieden werden. Nachfolgend werden die pharmazeutischen Zubereitungen kurz als "Lösemittel"und der Abbau kurz als "Auflösung" bezeichnet.

Der Wirkungsmechanismus der DSS ist noch nicht genau bekannt, es wird aber angenommen, daß die Substanzen über eine Spaltung der Disulfidbrücke der Mucoproteine wirken. Chemisch gesehen stellen die Disulfidbrückenspalter meist Thioalkohole (d.h. Thiole oder Mercaptane) dar, die eine SH-Gruppe tragen und dafür nachstehend kurz mit X-SH symbolisiert werden können. Vermutlich erfolgt beim Auflösen der organischen Matrix ein Angriff auf eine Disulfidbrücke —S-S—, der nach folgendem Schema abläuft :

$$A\text{-}S\text{-}S\text{-}B + X\text{-}SH \rightarrow A\text{-}SH + B\text{-}S\text{-}S\text{-}X \quad (1)$$

oder

$$A\text{-}S\text{-}S\text{-}B + X\text{-}SH \rightarrow B\text{-}SH + A\text{-}S\text{-}S\text{-}X \quad (2)$$

und den Abbau herbeiführt. Dabei bedeuten A und B über eine Disulfidbrücke verbundene organische Moleküreste. Chemisch gesehen handelt es sich bei den Reaktionen (1) und (2) um Redoxreaktionen.

Den die DSS oder Gemische davon enthaltenden Lösungen werden Komplexbildner zugesetzt, die zur Komplexierung von Calcium befähigt sind. Geeignete Komplexbildner sind z.B. Chelatkomplexbildner wie die Ethylendiamintetraessigsäure (EDTA) oder Salze davon. Bevorzugte EDTA-Salze sind die Alkalisalze, besonders bevorzugt ist das Dinatriumsalz (EDTA-Na$_2$). Nachfolgend wird unter der Kurzbezeichnung EDTA sowohl die freie Säure als auch ihre Salze verstanden, wenn nichts anderes angegeben ist. Daneben wird unter dem Kurzbegriff "Komplexbildner" allgemein ein Komplexbildner für Calciumatome verstanden.

Der Einsatz eines gemischten Wirkstoffs aus DSS + Komplexbildner eröffnet die Möglichkeit, die Zusammensetzung des Lösemittels optimierend an die Zusammensetzung des aufzulösenden Steines anzupassen. Liegt im Stein ein hoher Gehalt an organischer Matrix vor, wählt man einen hohen DSS-Gehalt im Lösemittel, bei hohem Calciumgehalt ist umgekehrt ein hoher Gehalt der Komplexbildner angezeigt. Die beiden Wirkstoffe Komplexbildner und DSS unterstützen einander dabei im Sinne eines synergistischen Effektes, d.h. das Gemisch aus beiden Wirkstoffen ergibt eine höhere Abbaugeschwindigkeit des Steins als jeder einzelne Wirkstoff für sich alleine. Dieser synergistische Effekt führt dazu, daß bei optimierter Zusammensetzung des Lösemittels bezüglich des Gehaltes an DSS und Komplexbildner die Auflösungszeit von Gallensteinen, insbesondere von Pigmentsteinen signifikant im Vergleich zu den bisher erreichten Zeiten verkürzt werden kann. Die Zusammensetzung des Lösemittels kann weiter dadurch optimiert werden, daß man mehr als einen DSS und/oder mehr als einen Komplexbildner einsetzt.

Weitere Zusätze dienen dazu, das herausgelöste Calciumbilirubinat (und auch Cholesterinanteile) kolloidal in Lösung zu halten bzw. zu emulgieren. Solche Emulgatoren lösen weitere synergistische Effekte in der Zusammenwirkung mit dem Komplexbildner und dem DSS aus. Es eignen sich alle Gallensäuren (natürliche Gallensäuren wie Cholsäure (CA), Desoxycholsäure (DCA), Chenodesoxycholsäure (CDCA), Glykocholsäure (GCA), Glykochenodesoxycholsäure (GCDCA), Ursodesoxycholsäure (UDCA), Taurocholsäure (TCA), Taurodesoxycholsäure (TDCA), Taurochenodesoxycholsäure (TCDCA) und künstliche Gallensäuren wie CHAPS, 3-((3-Cholamidopropyl)-dimethylammonium)-1-propansulfonsäure und ihre Salze, wobei TCA, CDCA und CHAPS, sowie deren Natriumsalze bevorzugt sind. Geeignet sind weiter synthetische Detergenzien wie Pluronic® F68 (Polyoxyethylen/polyoxypropylen-Polymer) und Palmidrol® (N-2(-Hydroxyethyl)-palmitamid) (beide z.B. bei Serva Heidelberg unter den angegebenen Handelsnamen erhältlich). Die Gallensäure oder das Gemisch aus Gallensäuren bzw. deren Salzen kann dabei neben der Emulgatorwirkung auch teilweise die Funktion der Puffersubstanzen übernehmen.

Ein erfindungsgemäßes Lösemittel zur Auflösung von Gallensteinen, insbesondere von Pigmentgallensteinen, enthält deswegen als Wirkstoffkombination einen Gehalt mindestens eines Komplexbildners und einen Gehalt mindestens eines Emulgators und mindestens eines DSS. Als Wirkstoffkombination wird ein Gemisch aus N-Acetylcystein (NAC) oder D-Penecillamin (oder Gemischen davon), dem Dinatriumsalz der Ethylendiamintetraessigsäure (EDTA-Na$_2$) und Taurocholsäure (TCA), Chenodesoxycholsäure(CDCA), CHAPS oder deren Salzen oder Gemischen davon in wäßriger Lösung besonders bevorzugt.

Neben den Wirkstoffkombinationen (d.h. dem DSS, dem Komplexbildner und dem Emulgator) müssen dem Lösemittel Puffersubstanzen zugefügt werden, die für die Einhaltung eines physiologisch tolerierbaren pH-Wertes sorgen. Eine solche Pufferung ist auch dann erforderlich, wenn ein Komplexbildner zugesetzt wird, der nur in einem bestimmten pH-Bereich arbeitet. Im Falle von EDTA-Na$_2$ liegt dieser Bereich bei etwa pH = 8 bis 9,5, wobei der Wert 9,5 die physiologische Unbedenklichkeitsgrenze darstellt. Geeignete Puffersubstanzen sind z.B. Aminosäuren wie Carnosin oder Arginin oder anorganische Puffergemische wie z.B. Borat/Carbonat.

Die Applikation der erfindungsgemäßen Lösemittel erfolgt bevorzugt durch direktes Einbringen der Lösemittel in die Gallenwege, um die aufzulösenden Steine mit dem Lösemittel zu umspülen. Dies kann z.B. mit einer über den Magen-Darm-Kanal in die Gallenwege eingeführten Sonde oder durch einen Katheter geschehen, der durch die Leber in die Gallenblase gelegt wird. Die Dosierung kann dabei z.B. 0,1 bis 10 ml/h betragen, wobei eine Dosierung im Bereich von 2 bis 5 ml/h bevorzugt ist.

Die Herstellung eines erfindungsgemäßen Lösemittels erfolgt durch Lösen der Wirkstoffe, der Puffersubstanzen und der Emulgatoren in Wasser und Einstellen ggf. des pH-Wertes der entstehenden Lösung auf den gewünschten pH-Wert mit Lauge (z.B. konzentrierter NaOH oder KOH). Dabei liegt die Konzentration des mindestens einen DSS bevorzugt im Bereich von 0,1 bis 40 Gew.-%, besonders bevorzugt im Bereich von 0,5 bis 2 Gew.-%. Der bevorzugte Konzentrationsbereich des mindestens einen Komplexbildners beträgt 0, 1 bis 20 Gew.-%, besonders bevorzugt 0,5-2 Gew.-%. Der bevorzugte Konzentrationsbereich des mindenstens einen Emulgators beträgt 0,1-5 Gew.-%, besonders bevorzugt 0,5-2 Gew.-%. Die Konzentration der Puffersubstanzen richtet sich nach dem gewünschten pH-Wert und der gewünschten Pufferkapazität des Lösemittels, die ihrerseits wieder von der DSS-Konzentration und der Komplexbildnerkonzentration abhängen. Unter Pufferkapazität wird dabei die Menge an 0,1 molarer HCl verstanden, die zu 10 ml des Lösemittels gegeben werden muß, um eine Absenkung des pH-Wertes um 1 zu erreichen. Bevorzugt sollte die Pufferkapazität bei einem Ausgangs-pH-Wert von 8 bis 9,5 (besonders bevorzugt 8,5-9) mindestens 1 ml der erwähnten HCl betragen, besonders bevorzugt mehr als 4 ml. Bei Einsatz von Aminosäuren und Gemischen davon als Puffersubstanzen wird dies mit Aminosäurekonzentrationen im Bereich von 0,5-12 Gew.-% erreicht, Borat/Carbonat-Puffer wird mit 2 mol/l eingesetzt.

Alle angegebenen Gewichtsprozente beziehen sich auf das Gesamtgewicht des Lösemittels.

Die nachfolgenden Beispiele erläutern die Erfindung weiter.

## I. Allgemeine Beschreibung der Auflösungsversuche

### 1. Steinmaterial

Pigmentgallensteine (PS) wurden von 12 Patienten gewonnen, an denen entweder eine Cholezystektomie (n = 8) oder eine endoskopische Papillotomie (n = 4) durchgeführt worden war. Die Steine wurden mit entionisiertem Wasser gewaschen, in einem Vakuumexsikkator getrocknet und in einem Achatmörser sorgfältig pulverisiert. Aliquote Teile davon wurden IR-spektroskopisch halbquantitativ vermessen. Steinpulver mit ähnlichen IR-Spektren und gleicher Lokalisation (Gallenblase oder Ductus choledochus)wurden gepoolt, in einem Achatmörser sorgfältig gemahlen und quantitativ auf Bilirubin, Cholesterin, Calciumsalze von Fettsäuren und Gesamtcalcium untersucht (Methode : Wosiewitz U, Schroebler S. : On the chemistry of "black" pigment stones from the gallbladder. Clin. Chim. Acta 1978, 89, 1-12 ; Wosiewitz U et al : Investigations on common bile duct stones. Digestion 1983, 26, 43-52). Für die Auflösungsversuche wurden unterschiedliche Proben eingesetzt :

a : gepooltes Pulver aus braunen Bilirubinatsteinen (gewonnen entweder aus der Gallenblase oder dem Ductus choledochus),

b : gepooltes Pulver aus schwarzen Polyrubinatsteinen (Gallenblase).

Tabelle I zeigt die Zusammensetzung der Proben a und b (Werte in Gew.-%, "Ca" bedeutet Calcium).

### 2. Auflösungsversuche

5-20 mg getrocknetes Pulver der Proben a und b wurden in kleine (1,5 ml) Reaktions- und Zentrifugierkapseln aus PTFE (Eppendorf) eingewogen. In jede Kapsel wurde 1 ml des zu erprobenden Lösemittels einpipettiert und die Kapsel 24 Stunden lang bei Raumtemperatur und abgedunkeltem Licht sanft geschüttelt. Anschließend wurden die Kapseln 3 Minuten lang bei 10500 xg zentrifugiert.

### 3. Ergebnisermittlung

a) Unmittelbar nach dem Zentrifugieren wurde der partielle Auflösungseffekt bestimmt, der auf in Lösung gegangenes Calciumbilirubinat zurückzuführen ist. Dazu wurden aliquote Teile des klaren Lysats spektrophotometrisch bei 436 nm gemessen (Filterphotometer von Eppendorf). Der Auflösungseffekt wurde berechnet als $\mu g$ aufgelöstes Calciumbilirubinat pro mg Feststoff (PS-Pulver), wobei für den molaren Extinktionskoeffizienten für Bilirubinat bei 436 nm der Wert von $55 \times 10^3$ mol $\times$ l$^{-1}$ $\times$ cm$^{-1}$ verwendet wurde.

b) Die Bestimmung des gesamten Auflösungseffektes erfolgte gravimetrisch durch Messung des Gewichtsverlustes einer jeden Probe nach dem Versuch. Dazu wurden die einzelnen Proben quantitativ abgesaugt und durch vorgewogene Membranfilterscheiben filtriert (Cellulosenitrat, Porenweite 8 μm, Durchmesser 25 mm). Nach der Filtration wurde der Rückstand jeweils gründlich mit deionisiertem Wasser gewaschen, um alle löslichen, aus dem Lösemittel stammenden Bestandteile zu entfernen. Danach wurden die Filterscheiben in einem Vakuumexsikkator über Phosphorpentoxid bis zur Gewichtskonstanz getrocknet.

c) Statistische Auswertung :

Alle nachfolgend in den Tabellen II-IV angegebenen Werte stellen Mittelwerte ($n \geq 5$) mit den jeweiligen Standardabweichungen dar. Für die abgegebenen p-Werte wurde der t-Test nach Student durchgeführt.

4. Bestandteile der Lösemittel

Bei den nachfolgend mitgeteilten Beispielen wurden folgende Lösemittelbestandteile verwendet :

a) Komplexbildner

Dinatriumsalz der Ethylendiamintetraessigsäure = EDTA

b) Disulfidbrückenspalter

N-Acetylcystein = NAC

c) Emulgatoren

Natriumtaurocholat = NaTCA und

Natriumsalz von 3-((3-Cholamidopropyl)-dimethylammonium)-1-propansulfonsäure = NaCHAPS

d) Puffersysteme

0,2 mol/l Borat/Natriumcarbonatpuffer, pH = 9,1-9,2 mit einem Zusatz von 10 mg/ml Ascorbat als Oxidationshemmer für Bilirubin.

II. Beispiele

Beispiele 1-12 (Probe a)

Die Beispiele betreffen Versuche mit Probe a (Pulver aus braunen Bilirubinatpigmentsteinen). Die Ergebnisse sind in Tabelle II zusammengestellt ; es sind jeweils der partielle Auflösungseffekt gemäß Punkt I.3a (in Lösung gegangenes Calciumbilirubinat = CaBR) und der gesamte Auflösungseffekt gemäß I.3b angegeben.

Erläuterungen zu Tabelle II :

| | |
|---|---|
| Beispiel 1 : | Auflösungseffekt des reinen Puffers |
| Beispiel 2-5 : | Auflösungseffekt mit nur einer dem Puffer zugesetzten Komponente (Komplexibildner, Emulgator oder DSS) |
| Beispiele 6-10 : | Auflösungseffekt mit zwei dem Puffer zugesetzten Komponenten (Komplexbildner + Emulgator, Komplexbildner + DSS oder Emulgator + DSS) |
| Beispiel 11 und 12 : | Auflösungseffekt mit drei dem Puffer zugesetzten Komponenten (Komplexbildner + Emulgator + DSS) |

Der synergistische Effekt beim Zusammenwirken von Komplexbildner und Emulgator ist z.B. durch Vergleich der Beispiele 2-4 mit den Beispielen 6 und 7 zu erkennen. Reines EDTA hat ein partielles Auflösungsvermögen von 168, die reinen Emulgatoren NaTCA und NaCHAPS zeigen Werte von 31,1 bzw. 42,8. Beim Zusammenwirken von EDTA und dem jeweiligen Emulgator erhält man Werte von etwa 400. Dies bedeutet gegenüber EDTA eine Steigerung um den Faktor 2,4. Ein weiterer synergistischer Effekt wird beim Zusammenwirken von NAC mit EDTA/NaTCA bzw. EDTA/NaCHAPS ausgelöst (Vergleich der Beispiele 5 und 6 mit den Beispielen 11 und 12 ; Steigerung von ca 400 auf ca 540 entsprechend einem weiteren Faktor von 1,35 bzw. einem Faktor von 3,2 gegenüber reinem EDTA).

Beispiele 13-16 (Probe b)

Die Beispiele betreffen Versuche mit Probe b (Pulver aus schwarzen Polybilirubinatpigmentsteinen). Die Ergebnisse sind in Tabelle III zusammengestellt (siehe Hinweise zu Beispiel 1-12).

Erläuterungen zu Tabelle III :

Beispiel 13 :     Auflösungseffekt des reinen Puffers

Beispiel 14 : Auflösungseffekt mit dem Puffer zugesetztem Komplexbildner (EDTA)

Beispiel 15 : Auflösungseffekt von mit dem Puffer zugesetztem Komplexbildner (EDTA) + Emulgator (NaTCA)

Beispiel 16 : Auflösungseffekt mit dem Puffer zugesetztem Komplexbildner (EDTA) + Emulgator (NaTCA) + DSS (NAC)

Der synergistische Effekt am Zusammenwirken des Komplexbildners mit dem Emulgator wird beim Vergleich der Beispiele 14 und 15 min Beispiel 16 deutlich (Faktor gegenüber EDTA = 2,9).

Tabelle I: Stein- und Probenmaterial

| Bestandteil | Probe a Gallen-blase | Ductus choled. | Probe b Gallen-blase | Probe c Gallen-blase |
|---|---|---|---|---|
| Cholesterin | 10,5 | 7,2 | 2,2 | - |
| Ca-Salze von Fettsäuren | 3,4 | 8,5 | - | - |
| Ca-Bilirubinat | 45,9 | 44,1 | 8,9 | 5,4 |
| Gesamt-Ca | 4,2 | 3,2 | 7,2 | 2,2 |

Tabelle II: Auflösung von Bilirubinpigmentstein-Material (Probe a) mit Lösemitteln unterschiedlicher Zusammensetzung nach 24stündigem Schütteln bei pH=9,2 (CaBR bedeutet Calciumbilirubinat)

| Zusammensetzung des Lösemittels | µg CaBR/mg Feststoff | Gesamter Gewichts-verlust, % |
|---|---|---|
| 1  0.2M Puffer | 13.7 ± 0.7 | 10.0 ± 1.2 |
| 2  -"- + 1% EDTA | (A)168.0 ± 9.8 | (a) 45.8 ± 1.9 |
| 3  -"- + 1% NaTCA | 31.1 ± 3.9 | 23.5 ± 3.2 |
| 4  -"- + 1% NaCHAPS | 42.8 ± 7.8 | 21.7 ± 1.9 |
| 5  -"- + 2% NAC | 18.8 ± 1.4 | 10.7 ± 1.5 |
| 6  -"- + 1%EDTA,1%NaTCA | (B)397.7 ±58.8 | (b) 64.3 ± 6.8 |
| 7  -"- + 1%EDTA,1%NaCHAPS | (C)398.9 ±26.0 | (c) 71.2 ± 3.9 |
| 8  -"- + 1%EDTA,2%NAC | 181.2 ± 4.4 | 50.4 ± 5.7 |
| 9  -"- + 1%NaTCA,2%NAC | 35.1 ± 5.3 | 23.7 ± 3.1 |
| 10  -"- + 1%NaCHAPS,2%NAC | 37.6 ± 7.2 | 18.4 ± 1.8 |
| 11  -"- + 1%EDTA,1%NaTCA,+ 2%NAC | (D)537.7 ±23.5 | (d) 82.4 ± 0.6 |
| 12  -"- + 1%EDTA,1%NaCHAPS, + 2%NAC | (E)538.3 ±16.6 | (e) 84.0 ± 1.2 |

$p <$ 0.0001 bei Vergleich von d und e mit a

$p <$ 0.001  bei Vergleich von c mit a und von B und C mit A

$p <$ 0.01   bei Vergleich von b mit a, d mit b, e mit c, D mit B und E mit C.

Tabelle III: Auflösung von Polybilirubinatpigmentstein-Material
(Probe b ) mit Lösemitteln unterschiedlicher Zusammensetzung nach
24stündigem Schütteln bei pH= 9,2 (CaBR bedeutet Calciumbilirubinat)

| Zusammensetzung des Lösemittels | $\mu$g CaBR/mg Feststoff | Gesamter Gewichtsverlust,% |
|---|---|---|
| 13 0.2M Puffer | 11.1 $\pm$ 0.5 | 18.7 $\pm$ 3.0 |
| 14 -"- + 1% EDTA | 16.3 $\pm$ 0.6 | 31.5 $\pm$ 3.0 * |
| 15 -"- + 1% EDTA,1%NaTCA | 47.3 $\pm$ 4.9 | 40.8 $\pm$ 6.0 * |
| 16 -"- + 1% EDTA,1%NaTCA,2%NAC | 36.4 $\pm$ 3.8 | 35.4 $\pm$ 2.6 |

* p < 0.001 bei Vergleich mit den Werten des reinen Puffers.

**Patentansprüche**

1. Pharmazeutische Zubereitung aus
a) Wasser
b) Puffersubstanzen
c) mindestens einem zur Komplexierung von Calcium befähigten Komplexbildner und
d) mindestens einem Emulgator, dadurch gekennzeichnet, daß sie zusätzlich noch mindestens einen Disulfidbrückenspalter (e) aufweist.

2. Zubereitung nach Anspruch 1, dadurch gekennzeichnet, daß der mindestens eine Komplexbildner (c) EDTA oder EDTA-Na$_2$ ist.

3. Zubereitung nach einem der Ansprüche 1 oder 2, dadurch gekennzeichnet, daß der mindestens eine Emulgator (d) eine künstliche oder natürliche Gallensäure oder ein Salz davon ist.

4. Zubereitung nach einem der Ansprüche 1-3, dadurch gekennzeichnet, daß der mindestens eine Disulfidbrückenspalter (e) eine Verbindung aus der Gruppe Cystein, N-Acetylcystein (NAC), Cysteamin, D-Penecillamin (DPA), Mercaptopropionglycin, Dithiothreitol, Glutathion (GSH), Methionin und anorganischen Sulfiden oder Hydrogensulfiden des Natriums, Kaliums oder Ammoniums ist.

5. Zubereitung nach einem der Ansprüche 1-4, dadurch gekennzeichnet, daß sie

Borat/Carbonat als Puffer (b),
EDTA oder EDTA-Na$_2$ als Komplexbildner (c),
Taurocholsäure (TCA), Chenodesoxycholsäure (CDCA),
CHAPS, deren Salze oder Gemische davon als Emulgator (d), und
N-Acetylcystein (NAC) oder D-Penecillamin (DPA) oder Gemische davon als Disulfidbrückenspalter (e) enthält.

6. Zubereitung nach Anspruch 5, gekennzeichnet durch folgenden Gehalt, jeweils bezogen auf das Gesamtgewicht der Zubereitung :

0,1 bis 0,3 mol/l Borat/Carbonat als Puffer (b),
0,5 bis 2 Gew.-% EDTA-Na$_2$ als Komplexbildner (c),

0,5 bis 2 Gew.-% Natriumtaurocholat (NaTCA) oder NaCHAPS als Emulgator (d), und
0,5 bis 2 Gew.-% N-Acetylcystein als Disulfidbrückenspalter (e), in Wasser.

7. Verwendung der pharmazeutischen Zubereitung nach einem der Ansprüche 1 bis 6 zur Herstellung von Mitteln zur Cholelitholyse, insbesondere zur Auflösung von Pigmentgallensteinen.

## Claims

1. Pharmaceutical preparation composed of
a) water
b) buffer substances
c) at least one complexing agent capable of complexation of calcium and
d) at least one emulsifier,
characterised in that it also has in addition at least one disulphide bridge splitter (e).

2. Preparation according to Claim 1, characterised in that the at least one complexing agent (c) is EDTA or EDTA-Na$_2$.

3. Preparation according to one of Claims 1 or 2, characterised in that the at least one emulsifier (d) is an artificial or natural bile acid or a salt thereof.

4. Preparation according to one of Claims 1-3, characterised in that the at least one disulphide bridge splitter (e) is a compound from the group comprising cysteine, N-acetylcysteine (NAC), cysteamine, D-penicillamine (DPA), mercaptopropionylglycine, dithiothreitol, glutathione (GSH), methionine and inorganic sulphides or hydrosulphides of sodium, potassium or ammonium.

5. Preparation according to one of Claims 1-4, characterised in that it contains

borate/carbonate as buffer (b),
EDTA or EDTA-Naz as complexing agent (c),
taurocholic acid (TCA), chenodeoxycholic acid (CDCA),
CHAPS, their salts or mixtures thereof as emulsifier (d),
and
N-acetylcysteine (NAC) or D-penicillamine (DPA) or mixtures thereof as disulphide bridge splitter (e).

6. Preparation according to Claim 5, characterised by the following content, in each case based on the total weight of the preparation :

0.1 to 0.3 mol/l borate/carbonate as buffer (b),
0.5 to 2% by weight EDTA-Na$_2$ as complexing agent (c),
0.5 to 2% by weight sodium taurocholate (NaTCA) or
NaCHAPS as emulsifier (d), and
0.5 to 2% by weight N-acetylcysteine as disulphide bridge splitter (e), in water.

7. Use of the pharmaceutical preparation according to one of Claims 1 to 6 for preparing agents for cholelitholysis, in particular for dissolving pigment gallstones.

## Revendications

1. Préparation pharmaceutique contenant :
a) de l'eau,
b) des substances tampons,
c) au moins un agent complexant apte à la complexation du calcium, et
d) au moins un émulsifiant,
caractérisée en ce qu'elle contient en outre au moins un agent de séparation (e) de ponts disulfures.

2. Préparation selon la revendication 1, caractérisée en ce que l'agent complexant minimal (c) est l'EDTA ou l'EDTA-Na$_2$.

3. Préparation selon la revendication 1 ou 2, caractérisée en ce que l'émulsifiant minimal (d) est un acide biliaire synthétique ou naturel ou un sel de celui-ci.

4. Préparation selon l'une des revendications 1 à 3, caractérisée en ce que l'agent de séparation minimal (e) de ponts disulfures est un composé choisi dans le groupe comprenant la cystéine, la N-acétylcystéine (NAC), la cystéinamine, la D-pénicillamine (DPA), la mercaptopropionglycine, le dithiothreitol, le glutathion (GSH), la méthionine et des sulfures ou hydrogénosulfures inorganiques du sodium, du potassium ou de l'ammonium.

5. Préparation selon l'une des revendications 1 à 4, caractérisée en ce qu'elle contient :
— du borate/carbonate comme tampon (b),
— de l'EDTA ou de l'EDTA-Na$_2$ comme agent complexant (c),
— de l'acide taurocholique (TCA), de l'acide chénodésoxycholique (CDCA), un CHAPS, des sels ou mélanges de ces composés, comme émulsifiant (d), et
— de la N-acétylcystéine (NAC) ou de la D-pénicillamine (DPA) ou des mélanges de ces composés, comme séparateur (e) de ponts disulfures.

6. Préparation selon la revendication 5, caractérisée en ce qu'elle contient les quantités suivantes, exprimées respectivement par rapport au poids total de la composition :

0,1 à 0,3 mol/1 de borate/carbonate comme tampon (b),
0,5 à 2% en poids d'EDTA-Na$_2$ comme agent complexant (c),
0,5 à 2% en poids de taurocholate de sodium (NaTCA) ou de NaCHAPS comme émulsifiant (d), et
0,5 à 2% en poids de N-acétylcystéine comme séparateur (e) de ponts disulfures, dans de l'eau.

7. Utilisation de la préparation pharmaceutique selon l'une des revendications 1 à 6, pour préparer des agents de cholélitholyse, en particulier pour dissoudre des calculs biliaires pigmentaires.